# EUROPEAN PATENT SPECIFICATION

(11) **EP 1 871 485 B1**
(45) Date of publication and mention of the grant of the patent: **23.04.2014**
(21) Application number: 06738796.9
(22) Date of filing: 15.03.2006
(51) Int. Cl.: A61Q 5/00, A61K 8/99, A23L 1/30, A61Q 3/00, A23L 1/302, A23L 1/304, A23L 1/308

(54) **USE OF AN ORALLY ADMINISTERED DIETARY SUPPLEMENT FOR REGULATING THE COSMETIC APPEARANCE OF HUMAN KERATINOUS TISSUE**
VERWENDUNG EINER ORAL VERABREICHTEN NAHRUNGSERGÄNZUNG ZUR REGULIERUNG DER KOSMETISCHEN ERSCHEINUNG MENSCHLICHEN KERATIN-GEWEBES
UTILISATION D'UN COMPLEMENT ALIMENTAIRE ADMINISTRE PAR VOIE ORALE POUR REGULER L'ASPECT COSMETIQUE DU TISSU KERATINEUX HUMAIN

(30) Priority: 29.03.2005 US 666029 P
(43) Date of publication of application: 02.01.2008
(73) Proprietor: The Procter & Gamble Company, Cincinnati, OH 45202 (US)
(72) Inventor: TREJO, Amy, Violet, Oregonia, Ohio 45054 (US)
(74) Representative: Hirsch, Uwe Thomas M.H.
(86) International application number: PCT/US2006/009779
(87) International publication number: WO 2006/104730

(56) References cited:
- EP-A- 1 609 463
- EP-A- 1 642 570
- WO-A-03/070203
- WO-A-03/070260
- WO-A-2005/030230
- WO-A2-2004/068970
- US-A1- 2005 123 500
- ISOLAURI E ET AL: "PROBIOTICS IN THE MANAGEMENT OF ATOPIC ECZEMA" November 2000 (2000-11), CLINICAL AND EXPERIMENTAL ALLERGY, BLACKWELL SCIENTIFIC PUBLICATIONS, LONDON, GB, PAGE(S) 1604-1610 , XP001064231 ISSN: 0954-7894 the whole document

## Description

### TECHNICAL FIELD

The present invention relates to regulating human keratinous tissue (e.g., hair, and/or nails). More particularly, the invention relates to regulating the appearance of human keratinous tissue by orally administering a dietary supplement comprising a probiotic to a human.

### BACKGROUND

Human keratinous tissue (e.g., skin, hair, and/or nails) is subject to insults by many extrinsic and intrinsic factors. Extrinsic factors include environmental pollution, wind, heat, low humidity, ultraviolet radiation (e.g., from sun exposure).

Intrinsic factors include chronological aging and other biochemical changes produced by the body. Additionally, diet and nutrition can have a major impact on the appearance of the hair, skin, and nails. In today's society, many people's diets are nutrient deficient and/or imbalanced, resulting in less than optimal keratinous tissue appearance. These extrinsic and intrinsic factors, as well as an insufficient diet, can contribute to hair appearance that is lifeless and dull, rather than attractive and shiny, skin that suffers from imperfections, and to unattractive nails that are brittle and break easily.

Thus, many consumers have keratinous tissue that is not as visually attractive and/or as healthy as desired. Accordingly, there is a need to provide consumers with a means of achieving healthier and/or more attractive keratinous tissue.

WO03070260 describes an orally administrable composition for the photoprotection of the skin which comprises a photoprotecting effective amount of at least one probiotic lactic acid bacterium or a culture supernatant thereof, and at least one yeast, included into an orally acceptable carrier.

WO03070203 describes an orally administrable composition for the photoprotection of the skin which comprises the combination of (i) at least one probiotic lactic acid bacterium or a culture supernatant thereof, and (ii) at least one carotenoid or derivative, included into an orally acceptable carrier.

Isolauri et al: "Probiotics in the management of atopic eczema" Clin Exp Allergy. 2000 Nov; 30(11):1604-1610 relates to administration of probiotics in the management of atopic eczema.

EP1642570 is prior art under Art 54(3) EPC and describes the use of a combination of Lactobacillus and Bifidobacterium species to prepare a dermatological composition for treatment and/or prevention of reactive, sensitive skin, optionally where associated with dry skin.

W02004/068970 describes a personal care kit comprising separate containers packaged together in a unitary form comprising a product suitable for oral consumption and a topical personal care product and a method of improving the condition of skin and/or hair by following a regimen utilizing the kit of the present invention.

EP1609463 is prior art under Art 54(3) EPC and describes the use of one or more probiotic microorganisms and/or their fractions and/or their metabolites in association with one or more divalent mineral cations other than sulfate to make compositions for oral administration to treat and/or prevent dry skin or compositions for application for the same purpose, the compositions themselves and a method of treatment using them.

### SUMMARY

The present invention provides consumers with a means of achieving more cosmetically attractive keratinous tissue (e.g., hair, and/or nails). In particular, the invention provides for the method of use of a dietary supplement by a human, for the purpose of regulating the cosmetic appearance of human keratinous tissue. More particularly, the invention relates to orally administering a dietary supplement comprising a probiotic and/or a culture supernatant thereof to a human. Still more particularly, it relates to orally administering a dietary supplement comprising a lactic acid bacterium and/or a culture supernatant thereof to a human. Preferably, the probiotic is administered in a safe and effective amount.

In another aspect, the present disclosure provides a method for informing a consumer that oral administration of the dietary supplement can regulate the attractiveness and/or health of the keratinous tissue. In still another aspect, it provides a method of marketing. In yet another aspect, the disclosure is directed to an article of commerce.

### DETAILED DESCRIPTION

While the specification concludes with the claims particularly pointing and distinctly claiming the invention, it is believed that the present invention will be better understood from the following description.

All percentages and ratios used herein are by weight of the total composition and all measurements made are at 25°C, unless otherwise designated.

The term "keratinous tissue," as used herein, refers to keratin-containing layers disposed as the outermost protective covering of humans, which includes, but is not limited to, hair, toenails, fingernails.

The terms "oral", "orally", and "oral administration", as used herein, refer to orally ingesting a composition in accordance with the present invention.

The term "orally acceptable", as used herein, means that the compositions or components thereof so described are suitable for oral ingestion by a human without undue toxicity, incompatibility, instability, allergic response.

As used herein, "effective amount" means an amount of a compound or composition sufficient to significantly induce a positive keratinous tissue benefit, preferably a positive keratinous tissue appearance, health, kinesthetic, or feel benefit, either independently or in combination with other benefits. This means that the content and/or concentration of probiotic in the formulation is sufficient that when the composition is administered with normal frequency and in a normal amount, the composition can result in the regulation of one or more undesired keratinous tissue conditions (e.g., skin wrinkles). For instance, the amount can be an amount sufficient to inhibit or enhance some biochemical function occurring within the keratinous tissue. This amount of probiotic may vary depending upon the type of product, the type of keratinous tissue condition to be addressed.

The term "safe and effective amount" as used herein means an amount of a compound or composition sufficient to significantly induce a positive benefit, preferably a positive keratinous tissue appearance, health, kinesthetic, or feel benefit, either independently or in combinations with other benefits, but low enough to avoid serious side effects, i.e., to provide a reasonable benefit to risk ratio, within the scope of sound judgment of the skilled artisan.

The dietary supplements in the use of the present invention can be useful for regulating keratinous tissue (e.g., hair, or nails) condition. As use herein, "regulating" or "regulation" means maintaining or improving the cosmetic appearance, and includes regulating visual, tactical, and kinesthetic properties of keratinous tissue, and can include prophylactically regulating and/or therapeutically regulating. Regulation of keratinous tissue condition, in particular human hair, or nail condition, is often required due to conditions which may be induced or caused by factors internal and/or external to the body. Examples include environmental damage, radiation exposure (including ultraviolet radiation), chronological aging, menopausal status (e.g., postmenopausal changes in hair, or nails), stress, diseases, disorders. For instance, "regulating hair, or nail condition" includes prophylactically regulating and/or therapeutically regulating hair, or nail condition, and may involve one or more of the following benefits: thickening of hair, or nails (e.g., where applicable the keratinous layers of the nail and hair shaft) to reduce hair, or nail atrophy, increasing the convolution of the dermal-epidermal border (also known as the rete ridges), preventing loss of hair elasticity such as loss of hair recoil from deformation; melanin or non-melanin change in coloration to the hair, or nails such as graying hair. Regulating can include delaying, minimizing, preventing, ameliorating, and/or effacing undesired keratinous tissue conditions which may be detected visually, by feel, or otherwise. For instance, regulating can include regulating the signs of aging.

The terms "smoothing" and "softening" as used herein mean altering the surface of the keratinous tissue such that its tactile feel is improved.

The terms "signs of keratinous tissue aging" or "signs of aging" include, but are not limited to, all outward visibly and tactilely perceptible manifestations as well as any other macro or micro effects due to keratinous tissue aging. Such signs may be induced or caused by intrinsic factors or extrinsic factors, e.g., chronological aging and/or environmental damage.

As used herein, the term "probiotic" is broad enough to include one or more probiotics, one or more culture supernatants thereof, and/or mixtures thereof.

The dietary supplements for use in the present invention are described in detail hereinafter.

### A. USE OF DIETARY SUPPLEMENT FOR THE PURPOSE OF REGULATING

### THE APPEARANCE OF HUMAN KERATINOUS TISSUE

According to the present invention, the dietary supplement is suitable for oral consumption and is administered orally. In preferred embodiments, the dietary supplement is in the form of capsules (e.g., those meant to be swallowed or chewed), tablets (e.g., those meant to be swallowed or chewed), powders (e.g., the powder can be added to water, milk, or another liquid), liquids (e.g., either in ready to drink form or suitable for dilution in another beverage), or nutritional foodstuffs. For example, the dietary supplement can be in the form of nutritional bars (e.g., meal or snack bars), cookies, candies (e.g., taffies, caramels, jellies, chocolate melt-aways, chews such as fruit chews, gums), syrups, or beverages (e.g., ready to drink or in the form of concentrates or powders).

As used herein, the term "dietary supplement" is broad enough to include not only single dosage forms, but also multiple dosage forms used in conjunction with one another. For instance, in one embodiment, the dietary supplement is a capsule (e.g., containing both probiotic lactic acid bacteria and a culture supernatant thereof). In another embodiment, the dietary supplement is two capsules (e.g., one containing probiotic lactic acid bacteria and one containing a culture supernatant thereof). In yet another embodiment, the dietary supplement comprises a beverage containing probiotic lactic acid bacteria that is consumed in conjunction with a capsule also containing probiotic lactic acid bacteria. As used here, "consumed in conjunction with" or "used in conjunction with" means orally consumed at the same time or at different times by the same user.

The regimen of consumption can vary according to the form of the dietary supplement. For instance, if the dietary supplement is in the form of a capsule or tablet, it is preferably taken orally with one or more meals daily. For example, the capsule or tablet may be taken daily with breakfast, lunch, and/or dinner. More preferably, there is oral consumption of the capsule or tablet form of dietary supplements in the use of the present invention over an extended period during the subject's lifetime, preferably daily. While benefits are obtainable after various maximum periods of use (e.g., five, ten or twenty years), it is preferred that oral consumption of the capsule or tablet form of dietary supplements in the use of the present invention continues throughout the subject's lifetime.

In combination with the oral consumption of the dietary supplements in the use of the present invention, a product suitable for topical application can optionally be applied to the hair, and/or nails as part of a combined treatment regimen. In one embodiment, one or more topical products are applied to the hair, and/or nails for the purpose of increasing their attractiveness, in addition to orally consuming the dietary supplement for the same purpose. In another embodiment, a combined treatment regimen is not used; rather, the dietary supplement is orally consumed but topical products are not also used for the purpose of improvement in hair, and/or nail appearance (e.g., increasing hair shine and/or decreasing nail breakage and/or brittleness).

In one embodiment, the dietary supplement is administered at least one time per day. In a particular embodiment, the dietary supplement is administered before the human goes to bed at night. In still another embodiment, the dietary supplement is administered every day, at night before retiring for bed. Preferably, the dietary supplement is administered at a safe and effective amount.

The dietary supplement herein comprises: (1) probiotic, and (2) optionally, one or more optional components.

### 1. Probiotic

Within the following description, "NCC" designates Nestle Culture Collection (Nestle Research Center, Vers-chez-les-Blanc, Lausanne, Switzerland).

According to the present invention, the subject compositions comprise, as the active agents therefor, at least one probiotic. In a particular embodiment, the probiotic comprises a probiotic lactic acid bacterium and/or a culture supernatant thereof.

Probiotics are non-pathogenic and non-toxigenic organisms that survive passage through the stomach and small intestine. Upon continuous ingestion by the host, they eventually may colonize the gut to a substantial extent thus competing with other potentially pathogenic bacteria for nutrients and/or attachment sites on the gastrointestinal wall and reducing their numbers and reducing or preventing infections.

A number of different probiotic microorganisms have been found, which are reported to exert their effect in the gut via the production of toxins, metabolic byproducts, short chain fatty acids. However, probiotics can also exert an effect in an individual's body at a location distant from the region in which they colonize it. For example, see WO 03/070203 Al, published August 28, 2003, which describes photoprotection resulting from the combined usage of carotenoids with lactic acid bacteria.

In one embodiment of the present invention, the probiotic selected from the group consisting of lactic acid bacteria, in particular Lactobacilli and/or Bifidobacteria, and more particularly Lactobacillus johnsonii, Lactobacillus reuteri, Lactobacillus rhamnosus, Lactobacillus paracasei, Lactobacillus casei or Bifidobacterium bifidum, Bifidobacterium breve, Bifidobacterium longum, Bifidobacterium animalis, Bifidobacterium lactis, Bifidobacterium infantis, Bifidobacterium adolescentis, Bifidobacterium pseudocatenulatum, or a mixture thereof. According to one embodiment the strains Lactobacillus johnsonii NCC 533, Lactobacillus paracasei NCC 2461, Bifidobacterium adolescentis NCC 251 and Bifidobacterium longum NCC 490 were deposited by way of an example, under the Budapest Treaty with the Institut Pasteur (28 rue du Docteur Roux, F-75024 Paris cedex 15) on June 30, 1992, January 12, 1999, April 15, 1999, and March 15, 1999, respectively and under the deposit number CNCM I-1225, CNCM I-2116, CNCM I-2168 and CNCM I-2170, 2 respectively. Other strains that can be used include the strain of Bifidobacterium lactis (ATCC27536) provided by Hansen (Chr. Hansen A/S, 10-12 Boege Alle, P.O. Box 407, DK-2970 Hoersholm, Danemark).

The probiotic microorganism according to the present invention may be included in a live form, dead form, semi-active or in deactivated form and fragments or fractions originating from the microorganism either live or dead (e.g., as a lyophilized powder). Most preferably, the probiotic comprises live probiotic microorganisms. Also culture supernatants of the microorganisms may be included in the products, optionally in concentrated form. It may also be included in an encapsulated form.

When using a supernatant of a probiotic's culture the supernatant may be used as such or may be subjected to one or more purification steps prior to inclusion into the product, so as to concentrate or isolate the active ingredient (s) /metabolite (s). Method and techniques for purifying compounds and detecting the activity thereof in the fractions obtained are well known to the skilled person.

In one embodiment, the probiotic can be present in the carrier in an amount of at least 10⁵ cfu/g of orally acceptable carrier, more specifically from about 10⁵ to about 10¹⁵ cfu/g of orally acceptable carrier, and still more specifically from about 10⁷ to about 10¹² cfu/g of orally acceptable carrier.

In one embodiment, the daily dose is from about 0.00001g to about 1g, more particularly from about 0.0001g to about 0.5g, and even more particularly from about 0.0005g to about 0.1g of probiotic bacteria.

### 2. Optional Components

### a. Orally acceptable carrier

The carrier may be any food or pharmaceutical product, or a nutritional supplement for oral administration or a composition for oral administration, wherein the probiotic microorganism may be included. Examples for food or pharmaceuticals carriers are milk, yogurt, curd, cheese, fermented milks, milk based fermented products, ice-creams, fermented cereal based products, milk based powders, infant formulae or tablets, liquid suspensions, dried oral supplement, wet oral supplement, dry-tube-feeding.

The nutritionally supplement for oral administration may be in capsules, soft capsules, tablets, pastes or pastilles, gums, or drinkable solutions or emulsions. Methods for preparing the carrier include those commonly known in the art. The composition according to the invention may further comprise bioactive molecules or yeast extracts, for example. In one embodiment, the yeast is any food-grade yeast selected from the group consisting of Ascomycotina or Deuteromycotina. In a particular embodiment, the yeast may be selected from the group consisting of Debaryomyces, Kluveromyces, Saccharomyces, Yarrowiu, Zygosaccharomyces, Candida, Rhodutorula, and/or Saccharomyces caerevisoe (baker's yeast). Such yeast may be used in the form of dried or lyophilized extracts. In one embodiment it may be present in the orally acceptable carrier in an amount of at least 10⁵ cfu/g of orally acceptable carrier, more specifically from about 10⁵ to 10¹⁵ cfu/g of orally acceptable carrier, and still more specifically from 10⁷ to 10¹² cfu/g of orally acceptable carrier, said amount depending on the nature and activity of the particular yeast.

### b. Prebiotic

The dietary supplement in the use of the present invention may also include a prebiotic. As used herein, the terms "prebiotic" and "prebiotics" are broad enough to include one or a mixture of more than one prebiotics. As used herein, "prebiotic" refers to a substrate on which bacteria (i.e., a probiotic) feeds. In one embodiment, the daily dose is from about 0.00001g to about 1g, more preferably from about 0.0001g to about 0.5g and, even more preferably, from about 0.0005g to about 0.1g, of the prebiotic.

Examples of prebiotics can include fiber, inulin, fructo-oligosaccharides, fructose, and mixtures thereof.

### c. Other Optional Components

The dietary supplement in the use according to the present invention can optionally include any suitable optional components. These optional components can include, but are not limited to, sugar amines (e.g., amino sugars), polyunsaturated fatty acids, anti-oxidants, vitamins, micronutrient metals, pro-biotics, pre-biotics, and combinations thereof. As used herein, the terms "optional component" and "optional components" are used interchangeably to mean one optional component or a mixture of more than one optional component.

### 1. Polyunsaturated Fatty Acids

The dietary supplement can include a safe and effective amount of polyunsaturated fatty acids. Polyunsaturated fatty acids are essentially polyethylenic acid (i.e., comprising at least two carbon-carbon double bonds). As used herein, the terms "polyunsaturated fatty acid" and "polyunsaturated fatty acids" are used interchangeably to mean one polyunsaturated fatty acid or a mixture of more than one polyunsaturated fatty acid. As used herein, the term "polyunsaturated fatty acids" is broad enough to include mono-, di-, and tri-glycerides, essential fatty acids, salts of fatty acids, and salts of essential fatty acids.

If used, preferably the polyunsaturated fatty acids used herein are essential fatty acids. As used herein, the terms "essential fatty acid" and "essential fatty acids" are used interchangeably to mean one essential fatty acid or a mixture of more than one essential fatty acid. As used herein, "essential fatty acids" refers to fats that are essential to the diet because the body cannot produce them and are classified as either omega-3 fatty acids or omega-6 fatty acids. If used, preferably, the daily dose is from at least about 0.1g to about 3g, more preferably from about 0.75g to about 2.5g and, even more preferably, from about 1g to about 2g, of the essential fatty acid.

Examples of omega-3 fatty acids that are useful herein include alpha-linolenic acid, stearidonic acid, eicosapentanoic acid, (EPA), docosahexanoic acid (DHA), and mixtures thereof. Preferably, the omega-3 fatty acid is EPA, DHA, or mixtures thereof.

Examples of omega-6 fatty acids that are useful herein include linoleic acid, gamma-linolenic acid, arachidonic acid, and mixtures thereof. Preferably, the omega-6 fatty acid is gamma-linolenic acid.

### 2. Anti-Oxidants

The dietary supplement in the use of the present invention can include a safe and effective amount of an anti-oxidant. As used herein, the terms "anti-oxidant" and "anti-oxidants" are used interchangeably to mean one anti-oxidant or a mixture of more than one anti-oxidant. If used, preferably, the daily dose is from about 0.00001g to about 1g, more preferably from about 0.0001g to about 0.5g and, even more preferably, from about 0.0005g to about 0.1g, of the anti-oxidant.

Anti-oxidants such as ester-C+, ascorbic acid (vitamin C) and its salts, ascorbyl esters of fatty acids, ascorbic acid derivatives (e.g., magnesium ascorbyl phosphate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox^{R}), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pidolate, nordihydroguaiaretic acid, bioflavonoids, lysine, methionine, proline, superoxide dismutase, silymarin, mixed carotenoids (i.e. beta-carotene, lutein, lycopene), tea extracts, grape skin/seed extracts, melanin, and rosemary extracts may be used. Preferred anti-oxidants are selected from tocopherol acetate and other esters of tocopherol, more preferably tocopherol acetate.

### 3. Vitamins

The dietary supplement in the use of the present invention can include a safe and effective amount of a vitamin. As used herein, the terms "vitamin" and "vitamins" are used interchangeably to mean one vitamin or a mixture of more than one vitamin. Furthermore, the terms "vitamin" or "vitamins" are broad enough as used herein to include pro-vitamins (e.g., beta-carotene). If used, preferably, the daily dose is from about 0.00001g to about 1g, more preferably from about 0.0001g to about 0.5g and, even more preferably, from about 0.0005g to about 0.1g, of the vitamin.

Examples of vitamins that are useful herein include vitamin A, vitamin B, vitamin C, vitamin D, vitamin E, vitamin K; their derivatives; and mixtures thereof.

### a) Vitamin B

Examples of Vitamin B compounds useful herein include vitamin B₁, vitamin B₂, vitamin B₃, vitamin B₅, vitamin B₆, vitamin B₁₂, vitamin B₁₅, their derivatives, and mixtures thereof.

For Example, a safe and effective amount of a vitamin B₃ compound may be used. When vitamin B₃ compounds are used, the daily dose is preferably from about 0.00001g to about 1g, more preferably from about 0.0001g to about 0.5g and, even more preferably, from about 0.0005g to about 0.1g of the vitamin B₃ compound.

As used herein, "vitamin B₃ compound" means a compound having the formula: 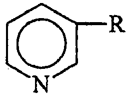 wherein R is - CONH₂ (i.e., niacinamide), - COOH (i.e., nicotinic acid) or - CH₂O (i.e., nicotinyl alcohol); derivatives thereof; and salts of any of the foregoing.

Exemplary derivatives of the foregoing vitamin B₃ compounds include nicotinic acid esters, including non-vasodilating esters of nicotinic acid (e.g., tocopheryl nicotinate), nicotinyl amino acids, nicotinyl alcohol esters of carboxylic acids, nicotinic acid N-oxide and niacinamide N-oxide.

Examples of suitable vitamin B₃ compounds are well known in the art and are commercially available from a number of sources, e.g., the Sigma Chemical Company (St. Louis, MO); ICN Biomedicals, Inc. (Irvin, CA) and Aldrich Chemical Company (Milwaukee, WI).

The vitamin compounds may be included as the substantially pure material, or as an extract obtained by suitable physical and/or chemical isolation from natural (e.g., plant) sources.

Also for example, a safe and effective amount of a vitamin B₆ compound may be used. The vitamin B₆ compounds useful herein include pyridoxine, esters of pyridoxine (e.g., pyridoxine tripalmitate), amines of pyridoxine (e.g., pyridoxamine), salts of pyridoxine (e.g., pyridoxine HCl) and derivatives thereof, including pyridoxamine, pyridoxal, pyridoxal phosphate, and pyridoxic acid. More preferably, the vitamin B₆ is selected from the group consisting of pyridoxine, esters of pyridoxine and salts of pyridoxine. Most preferably, the vitamin B₆ is pyridoxine HCl.

Vitamin B₆ can be synthetic or natural in origin and can be used as essentially as pure compounds or mixtures of compounds (e.g., extracts from natural sources or mixtures of synthetic materials). Vitamin B₆ is generally found in many foodstuffs, especially yeast, liver and cereals. As used herein, "vitamin B₆" includes isomers and tautomers of such and is commercially available from Sigma Chemical Co., St. Louis, MO.

When vitamin B₆ compounds are present in the instant invention, the daily dose is preferably from about 0,00001g to about 1g, more preferably from about 0,0001g to about 0.5g and, even more preferably, from about 0.0005g to about 0.1g, of the vitamin B₆ compound.

### 4. Micronutrient Metals

The dietary supplement in the use of the present invention may also include a micronutrient metal. As used herein, the term "micronutrient metal" and "micronutrient metals" are used interchangeably to mean one micronutrient metal or a mixture of more than one micronutrient metal. As used herein, "micronutrient metal" refers to a metal that provides nutrients to a mammalian body that are necessary for proper total nutrition. If used, preferably, the daily dose is from about 0.00001g to about 1g, more preferably from about 0.0001g to about 0.5g and, even more preferably, from about 0.0005g to about 0.1g, of micronutrient metal.

Examples of micronutrient metal that are useful herein include iron, zinc, selenium, copper, manganese; their derivatives; their salts; and mixtures thereof. Preferably, the micronutrient metal is copper, selenium, zinc and mixtures thereof.

### 5. Sugar Amines (Amino Sugars)

As used herein, "sugar amine" refers to an amine derivative of a six-carbon sugar. As used herein, the term "sugar amine" is broad enough to include not only sugar amines, but also pharmaceutically acceptable salts thereof. Furthermore, the terms "sugar amine" and "sugar amines" are used interchangeably to mean one sugar amine or a mixture of more than one sugar amine.

If used, preferably, the daily dose of sugar amine from about 0,1g to about 3g, more preferably from about 0.75g to about 2.5g, and still more preferably from about 1g to about 2g.

Examples of sugar amines that are useful herein include, but are not limited to, glucosamine, N-acetyl glucosamine, mannosamine, N-acetyl mannosamine, galactosamine, N-acetyl galactosamine; their derivatives; their salts; and mixtures thereof. Preferably, the sugar amine is glucosamine. Preferred glucosamines include N-acetyl glucosamine, glucosamine, and glucosamine hydrochloride. Additionally, combinations of two or more sugar amines may be used.

### B. METHOD FOR REGULATING HUMAN CONSUMER KERATINOUS TISSUE, METHOD OF MARKETING A DIETARY SUPPLEMENT, ARTICLE OF COMMERCE, AND PERSONAL CARE KIT

In another aspect, the present disclosure provides a method for regulating the condition of human keratinous tissue. The method comprises the steps of:
(1) making available to a human consumer an orally administered dietary supplement comprising a probiotic;
(2) communicating to said consumer that oral administration of said dietary supplement can regulate the consumer's keratinous tissue;
(3) acquisition of said orally administered dietary supplement by said consumer; and
(4) oral administration of said dietary supplement by said consumer.

In another aspect, the present disclosure provides a method of marketing a dietary supplement that can be used to regulate human keratinous tissue. The method comprises the steps of:
(1) making available to a human consumer an orally administered dietary supplement comprising a probiotic; and
(2) communicating to said consumer that oral administration of said dietary supplement can regulate the consumer's keratinous tissue.

In yet another aspect, the present disclosure provides an article of commerce. The article of commerce comprises:
(1) an orally administered dietary supplement comprising a probiotic;
(2) a container that contains said dietary supplement; and
(3) a message associated with said dietary supplement, wherein said message informs a human consumer that oral administration of said dietary supplement can regulate said consumer's keratinous tissue.

In still another aspect, the present disclosure can provide a personal care kit. The personal care kit comprises:
(1) a dietary supplement comprising a probiotic;
(2) a topical product, wherein said topical product is suitable for topical application to keratinous tissue; and
(3) optionally, a message that instructs the consumer to use the dietary supplement and the topical product as part of a regimen.

The dietary supplement can be made available to the consumer by any suitable means. For instance, it can be offered for sale at a retail outlet or for purchase through a catalogue or via the internet. Furthermore, any suitable means of communication can be used to communicate to the consumer that oral administration of the dietary supplement can regulate the consumer's keratinous tissue. For example, communication can be in the form of printed material (e.g. package label, print advertisement), an electronic message via the internet, or a broadcast message through television or radio. For example, the communication can inform the consumer that administration of the dietary supplement can improve hair health, hair shine, hair luster, or hair radiance, or can improve nail health, nail strength, or can reduce nail brittleness or breakage. In one embodiment, the communication informs the consumer that administration of the dietary supplement can reduce the signs of aging, fight the signs of aging, reverse the signs of aging, and/or prevent the signs of aging. In a particular embodiment, the communication informs the consumer that administration of the dietary supplement can improve the beauty and/or attractiveness of the consumer.

In a particular embodiment, the communication informs the consumer that oral administration of the dietary supplement can lead to prevention and/or correction of the functional disorders of the pilo-sebaceous unit of mammals, particularly humans, and more specifically for the treatment of oily and/or hyper-seborrheic scalp, thin hair, hyper-trichosis, and/or alopecia. In another embodiment, the communication informs the consumer that oral administration of the dietary supplement can increase the density of hair, especially human hair, and/or reduce the heterogeneity of their diameter and/or improve their growth and/or prevent and/or reduce and/or delay hair loss. In still another embodiment the communication informs the consumer that oral administration of the dietary supplement can increase the hair volume.

As used herein, the term "heterogeneity of hair diameter" refers to a significant variation in the hair diameter in a specific region of the scalp; some hair having a physiological diameter in the range of 100 um, and others, in the nearest proximity of those hairs, having a reduced diameter (e.g., thin hair). Thus, by "reducing heterogeneity of the diameter," it is meant increasing the diameter of thin hair. By "increasing the density," it is meant increasing the number of hairs per square centimeter of skin or scalp.

The consumer can acquire the dietary supplement by any suitable means. For instance, the consumer can purchase the dietary supplement, can obtain the dietary supplement from a purchaser of the dietary supplement, can obtain the dietary supplement as a free sample, or can obtain the dietary supplement as a gift with, or in conjunction with, the purchase of another item. As used herein, the term "consumer" means a human consumer, and is broad enough to include both purchasers and potential purchasers of the dietary supplement.

Any container from which the dietary supplement can be dispensed, presented, displayed, or stored is suitable. Suitable containers include, but are not limited to, bottles, boxes, bags, and pouches, tubes, and tottles.

The personal care kit can be in any suitable form. For instance, it can be in the form of two separate containers (e.g., one containing a dietary supplement and one containing a topical product) packaged together into one unitary package. It can also be two separate containers that are intended for use together.

Optionally, the kit comprises a message that instructs the consumer to use the dietary supplement and topical product as part of a regimen (e.g, to use both products as part of a health and/or beauty regime, schedule, treatment). For instance, the communication can communicate to a consumer that the dietary supplement and the topical product are each to be used daily (e.g., at same times or at different times), are each to be used at night. The message can also optionally inform the consumer that the regimen can regulate human keratinous tissue. For instance, the message can inform the consumer that the regimen can regulate the health and/or cosmetic appearance of human hair, skin, and/or nails.

The kit can comprise any suitable topical product. Topical products are those personal care compositions that are suitable for topical application to keratinous tissue. For instance, suitable topical products can include creams, gels, lotions, emulsions, colloids, solutions, suspensions, ointments, milks, sprays, capsules, tablets, liquids, sticks, solids, powders, compacts, pencils, spray-on formulations, brush-on formulations, cloths, wipes.

Non-limiting examples of topical products can include, without limitation, lipstick, mascara, rouge, foundation, blush, eyeliner, lipliner, lip gloss, facial or body powder, nail polish, mousse, sprays, styling gels, nail conditioner, bath and shower gels, shampoos, conditioners, cream rinses, hair dyes and coloring products, leave-on conditioners, sunscreens and sunblocks, lip balms, skin conditioners, cold creams, moisturizers, hair sprays, soaps, body scrubs, exfoliants, astringents, depilatories and permanent waving solutions, antidandruff formulations, antisweat and antiperspirant compositions, shaving, preshaving and after shaving products, moisturizers, deodorants, cold creams, cleansers, skin gels, and rinses.

Furthermore, the topical product can be applied topically through the use of a patch or other delivery device. Delivery devices can include, but are not limited to, those that can be heated or cooled, as well as those that utilize iontophoresis or ultrasound.

For instance, the topical product can be applied, for example, by applying a composition in the form of a skin lotion, clear lotion, milky lotion, cream, gel, foam, ointment, paste, emulsion, spray, conditioner, tonic, cosmetic, lipstick, foundation, nail polish, after-shave, which is intended to be left on the skin or other keratinous tissue (i.e., a "leave-on" composition). After applying the composition to the keratinous tissue (e.g., skin), it in one embodiment, it is left on for a period of at least about 15 minutes, more preferably at least about 30 minutes, even more preferably at least about 1 hour, even more preferably for at least several hours, e.g., up to about 12 hours. In one embodiment, the topical product is left on overnight. In another embodiment, the topical product is left on all day. Any part of the external portion of the face, hair, and/or nails can be treated, (e.g., face, lips, under-eye area, eyelids, scalp, neck, torso, arms, hands, legs, feet, fingernails, toenails, scalp hair, eyelashes, eyebrows.)

Any suitable method can be used to apply the topical product. For instance, the application of the topical product can be done using the palms of the hands and/or fingers or a device or implement (e.g., a cotton ball, swab, pad, applicator pen, spray applicator). Another approach to ensure a continuous exposure of the keratinous tissue to at least a minimum level of the topical product is to apply the compound by use of a patch applied, e.g., to the face. Such an approach is particularly useful for problem skin areas needing more intensive treatment (e.g., facial crows feet area, frown lines, under eye area, upper lip). The patch can be occlusive, semi-occlusive or non-occlusive, and can be adhesive or non-adhesive. The topical product can be contained within the patch or be applied to the skin prior to application of the patch. The patch can also include additional actives such as chemical initiators for exothermic reactions such as those described in PCT application WO 9701313, and in U.S. Patents numbered 5,821,250, 5,981,547, and 5,972,957 to Wu, et al. The patch can also contain a source of electrical energy (e.g., a battery) to, for example, increase delivery of the composition and active agents (e.g., iontophoresis). The patch can be left on the keratinous tissue for any suitable period of time. For example, a period of at least about 5 minutes, more preferably at least about 15 minutes, more preferably still at least about 30 minutes, even more preferably at least about 1 hour, even more preferably at night as a form of night therapy, or in another embodiment all day.

The topical product can comprise any suitable desired materials. For instance, such materials can be selected from the group consisting of sugar amines (e.g., N-acetylglucosamine), vitamin B3 compounds, sodium dehydroacetate, dehydroacetic acid and its salts, phytosterols, soy derivaties (e.g., equol and other isoflavones), niacinamide, phytantriol, farnesol, bisabolol, salicylic acid compounds, hexamidines, dialkanoyl hydroxyproline compounds, flavonoids, N-acyl amino acid compounds, retinoids (e.g., retinyl propionate), water-soluble vitamins, ascorbates (e.g., vitamin C, ascorbic acid, ascorbyl glucoside, ascorbyl palmitate, magnesium ascorbyl phosphate, sodium ascorbyl phosphate), particulate materials, sunscreen actives, anti-cellulite agents, butylated hydroxytoluene, butylated hydroxyanisole, their derivatives, and combinations thereof. Other examples include cationic polymers, conditioning agents (hydrocarbon oils, fatty esters, silicones), anti-dandruff agents, suspending agents, viscosity modifiers, dyes, nonvolatile solvents or diluents (water soluble and insoluble), pearlescent aids, foam boosters, surfactants, nonionic cosurfactants, pediculocides, pH adjusting agents, perfumes, preservatives, chelants, chelating agents, proteins, UV absorbers, pigments, other amino acids, and other vitamins.

For instance, topical products for use herein may comprise one or more vitamins and/or amino acids such as: water soluble vitamins such as vitamin B1, B2, B6, B 12, C, pantothenic acid, pantothenyl ethyl ether, panthenol, biotin, and their derivatives, water soluble amino acids such as asparagine, alanine, indole, glutamic acid and their salts, water insoluble vitamins such as vitamin A, D, E, and their derivatives, water insoluble amino acids such as tyrosine, tryptamine, and their salts.

Topical products may also contain one or more pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. 1. Names. The topical products may also contain antimicrobial agents which are useful as cosmetic biocides and antidandruff agents including: water soluble components such as piroctone olamine, water insoluble components such as 3,4,4'-trichlorocarbanilide (trichlosan), triclocarban and zinc pyrithione.

Furthermore, the topical product can comprise peptides, such as those disclosed in U.S. Patent No. 6,492,326, issued December 10, 2002, to Robinson et al. (e.g., pentapeptides such as lys-thr-thr-lys-ser, and derivatives thereof). A preferred pentapeptide derivative is palmitoyl-lys-thr-thr-lys-ser, available from Sederma, France. Another preferred optional dipeptide that can be used is carnosine. Other suitable peptides can include di-, tri-, tetra-, penta-, or hexa-peptides and/or derivatives thereof.

Any other suitable materials can also be included in the topical product, such as those ingredients that are conventionally used in given product types. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the topical product herein. Examples of these ingredient classes include, but are not limited to: abrasives, absorbents, aesthetic components such as fragrances, pigments, colorings/colorants, essential oils, skin sensates, astringents, etc. (e.g., clove oil, menthol, camphor, eucalyptus oil, eugenol, menthyl lactate, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents, (e.g. hydroquinone, kojic acid, ascorbic acid, magnesiuim ascorbyl phosphate, ascorbyl glucoside, pyridoxine), skin-conditioning agents (e.g. humectants and occlusive agents), skin soothing and/or healing agents and derivatives (e.g. panthenol, and derivatives such as ethyl panthenol, aloe vera, pantothenic acid and its derivatives, allantoin, bisabolol, and dipotassium glycyrrhizinate), skin treating agents (e.g. vitamin D compounds, mono-, di-, and tri-terpenoids, beta-ionol, cedrol), thickeners, and vitamins and derivatives thereof.

### EXAMPLES

### Example 1-Dietary Supplement

A dietary supplement comprising a 500 mg probiotic is prepared in the form of a capsule. The dietary supplement is intended for administration to a human in the amount of one capsule daily.

### Example 2- Article of Commerce

The dietary supplement of Example 1 is packaged in a bottle and offered for sale in a retail outlet. A television advertisement contains information that informs a consumer that oral administration of the dietary supplement can improve the shine, radiance, and luster of the consumer's hair.

### Example 3 - Article of Commerce

The dietary supplement of Example 1 is packaged in a pouch and offered for sale in a retail outlet. A print advertisement in a magazine contains information that informs a consumer that oral administration of the dietary supplement can decrease breakage and brittleness of the consumer's nails.

### Example 4- Article of Commerce

The dietary supplement of Example 1 is packaged in a pouch and offered for sale in a retail outlet. A print advertisement in a magazine contains information that informs a consumer that oral administration of the dietary supplement can improve the appearance of both the hair and nails by increasing the shine of the consumer's hair and decreasing breakage of the consumer's nails.

### Example 5- Article of Commerce

The dietary supplement of Example 1 is packaged in a pouch and offered for sale in a retail outlet. A print advertisement in a magazine contains information that informs a consumer that oral administration of the dietary supplement can help reduce the visible signs of aging.

## Claims

1. Use of an orally administered probiotic material for the purpose of regulating the cosmetic appearance of at least one human keratinous tissue selected from the group consisting of hair, toenails and fingernails.

2. The use of claim 1, wherein said probiotic material comprises live probiotic material.

3. The use of any of the preceding claims, wherein said probiotic material is combined in a dietary supplement with a prebiotic.

4. The use of any of the preceding claims, wherein said probiotic material is included in a dietary supplement which is administered nightly.

5. The use of any of the preceding claims, wherein said probiotic material is included in a dietary supplement which is administered daily.

## Patentansprüche

1. Verwendung eines oral verabreichten probiotischen Materials zum Zwecke der Regulierung des kosmetischen Erscheinungsbilds mindestens eines menschlichen keratinhaltigen Gewebes ausgewählt aus der Gruppe bestehend aus Haar, Zehennägeln und Fingernägeln.

2. Verwendung nach Anspruch 1, wobei das probiotische Material lebendes probiotisches Material umfasst.

3. Verwendung nach einem der vorstehenden Ansprüche, wobei das probiotische Material in einem Nahrungsergänzungsmittel mit einem prebiotischen Mittel kombiniert ist.

4. Verwendung nach einem der vorstehenden Ansprüche, wobei das probiotische Material in einem Nahrungsergänzungsmittel enthalten ist, das nachts verabreicht wird.

5. Verwendung nach einem der vorstehenden Ansprüche, wobei das probiotische Material in einem Nahrungsergänzungsmittel enthalten ist, das tagsüber verabreicht wird.

## Revendications

1. Utilisation d'un matériau probiotique administré par voie orale dans le but de réguler l'apparence cosmétique d'au moins un tissu kératinique humain choisi dans le groupe constitué des cheveux, des ongles des pieds et des ongles de la main.

2. Utilisation selon la revendication 1, dans laquelle ledit matériau probiotique comprend un matériau probiotique vivant.

3. Utilisation selon l'une quelconque des revendications précédentes, dans lequel ledit matériau probiotique est combiné dans un complément diététique à un prébiotique.

4. Utilisation selon l'une quelconque des revendications précédentes, dans laquelle ledit matériau probiotique est inclus dans un complément diététique qui est administré chaque soir.

5. Utilisation selon l'une quelconque des revendications précédentes, dans laquelle ledit matériau probiotique est inclus dans un complément diététique qui est administré quotidiennement.
